# EUROPEAN PATENT SPECIFICATION

(11) **EP 4 385 488 B1**
(45) Date of publication and mention of the grant of the patent: **17.12.2025**
(21) Application number: 23213649.9
(22) Date of filing: 01.12.2023
(51) Int. Cl.: A61K 8/34, A61K 8/41, A61K 8/42, A61Q 5/12

(54) **CONDITIONING COMPOSITION FOR KERATIN FIBERS, ESPECIALLY HUMAN HAIR**
KONDITIONIERUNGSZUSAMMENSETZUNG FÜR KERATINISCHE FASERN, INSBESONDERE FÜR MENSCHLICHES HAAR
COMPOSITION DE CONDITIONNEMENT POUR FIBRES KÉRATINIQUES, NOTAMMENT POUR LES CHEVEUX HUMAINS

(30) Priority: 02.12.2022 EP 22211004
(43) Date of publication of application: 19.06.2024
(73) Proprietor: Kao Corporation, Chuo-ku Tokyo 103-8210 (JP)
(72) Inventor: HOFFMANN, Martin, 64297 Darmstadt (DE); HELMI, Ala-Mutka, 64297 Darmstadt (DE); ENTZMINGER, Anne, 64297 Darmstadt (DE)
(74) Representative: Hoffmann Eitle

(56) References cited:
- WO-A1-2021/219547
- GB-A- 2 091 102
- JP-A- H03 223 208
- US-A1- 2011 150 810
- US-A1- 2012 034 182
- US-B1- 6 607 718
- DATABASE GNPD [online] MINTEL; 2 August 2019 (2019-08-02), ANONYMOUS: "Conditioner", XP093048689, retrieved from https://www.gnpd.com/sinatra/recordpage/6769383/ Database accession no. 6769383

## Description

### Field of the invention

The present invention is on a substantially anhydrous conditioning composition for keratin fibers, especially human hair which requires substantially less product application onto keratin fibers and therefore especially economical and has many advantageous properties with respect to the economics and environmental protection.

### Background of the invention

Hair conditioning compositions have been known and used for ages. They are used for detangling the hair and providing further additional properties. They are usually aqueous compositions based on cationic surfactants and may additionally comprise oily substances, polymers and others. They may be emulsions which are usually rinsed off after application and processing on the hair, and solutions and/or dispersions which are usually left on the hair and not rinsed off. Since the compositions are generally aqueous compositions, they comprise only certain amount of active and therefore requires relatively larger amount to be applied onto hair. This, furthermore, results in use of larger packaging materials mainly made of mixture of plastic materials which are often not recyclable and requires larger quantities to be transported which automatically means larger CO₂ emission. These compositions may be seen in several aspects uneconomical and environmentally disadvantageous. As clearly pointed out there are many aspects which should definitively be overcome for the sake of environment and to reduce or keep the promises that the temperature increase will not exceed 1.5°C on average over time.

There have been efforts to overcome the above referred problems mainly by providing leave in compositions as detangling and further improvement of keratin fibers properties, which has not found a broad acceptance by the users because of mainly high loading on the hair, which results in volume reduction and makes hair in a way more covered and may as well be sticky.

Presence of cationic surfactants provide certain level of softening to the keratin fibers. It is quite important for the consumer feeling the softness during rinsing off the composition form hair and afterwards in towel dry and finally in dry state. Currently available conditioners have some drawback and therefore there is a need for further products.

WO 2021/219547 discloses substantially anhydrous compositions comprising one or more polyol, one or more mono alcohol, one or more fatty amine and one or more carboxylic acids. The compositions are transparent and provide certain level of conditioning on hair which has not always been found satisfactory.

US 6 607 718 B1 discloses hair cosmetic compositions that are prepared by dissolving a component in a system rich in a polyhydric alcohol with hydroxypropyl cellulose as a thickener.

JP H03 223208 A discloses hair treatment compositions comprising a cationic surfactant, a nonionic or amphoteric surfactant, an oil or fat and a water-soluble alcohol.

GNPD [database online], Mintel, Record ID 6769383 discloses a conditioner comprising C13-15 alkane and ethylhexylglycerin.

### Summary of the invention

Accordingly, the first object of the present invention is a substantially anhydrous composition for keratin fibers especially for human hair comprising
- one or more polyol other than the glyceryl ether,
- one or more amine and/or quaternary ammonium surfactant,
- one or more fatty alcohol,
- one or more glyceryl ether according to the general structure 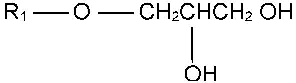
   wherein R₁ is straight or branched, saturated or unsaturated alkyl chain with 4 to 18 C atoms,
   wherein the composition comprises water not more than 10%, preferably 7.5%, more preferably 5% and most preferably 2.5% by weight, calculated to the total weight of the composition,
   wherein the composition comprises the one or more polyol at a concentration of at
   least 40% by weight, calculated to the total weight of the composition, and wherein the one or more glyceryl ether according to the above general structure is selected from ethylhexyl glycerin, hexylglycerin, heptylglycerin, methylheptylglycerin.

The term substantially anhydrous refers to the water content as given above. The composition comprises not more than 10% by weight of water. Preferably the composition does not comprise more than 7.5% by weight of water, more preferably not more than 5% by weight or water, and most preferably not more than 2.5% by weight of water, calculated to the total weight of the composition. The compositions are anhydrous in the particularly preferred form.

The second object of the present invention is a method of conditioning keratin fibers especially human hair, comprising the following steps:
(i) Treating the keratin fibers with an aqueous composition, preferably a cleansing composition, and then, when the keratin fibers are wet,
(ii) Applying the composition according to any of the claims 1 to 13 onto the keratin fibers and leaving it for a time period in the range of 30 s to 10 min, then
(iii) Rinsing off the composition from the keratin fibers, and then
(iv) Towel drying the keratin fibers.

The third object of the present invention is the use of the composition for conditioning keratin fibers, especially human hair, especially for improving softness, especially during application and rinsing off, surface smoothness, combability, shine and body.

Further object of the present invention is a method of conditioning keratin fibers especially human hair, comprising the following steps:
(i) Treating the keratin fibers with an aqueous composition, preferably a cleansing composition, and then, when the keratin fibers are wet,
(ii) Diluting the composition as defined above with water at a composition to water weight ratio of 1:1 to 1:10,
(iii) Applying the diluted composition onto the keratin fibers and leaving it for a time period in the range of 30 s to 10 min, then
(iv) Rinsing off the composition from the keratin fibers, and then
(v) Towel drying the keratin fibers.

Still further object of the present invention is a kit comprising two or more components wherein one of the components is a composition of the present invention.

### Detailed description of the invention

The inventors of the present invention have surprisingly and unexpectedly found that a substantially anhydrous composition comprising one or more polyols, one or more amine or quaternary ammonium surfactants, one or more fatty alcohols and one or more glyceryl ethers provides good conditioning of the hair in terms of softness, especially during application and rinsing, i.e. improves the conditioning effect on wet hair such as surface smoothness, combability, shine and body, even when a considerably small amount of product is applied to the hair. The hair is rinsed after the product has been applied, evenly distributed and left on the hair for a short period of time, preferably from 30 seconds to 10 minutes, followed by towel-drying and airdrying or blow-drying the hair.

The composition of the present invention comprises one or more polyol other than glyceryl ether. The term polyol refers to liquid compounds having two or more OH groups in the molecule. The glyceryl ethers which additionally required for the present invention is not counted as the polyol. The one or more polyol comprised in the compositions at a total concentration of at least 40%, preferably at least 50%, more preferably in the range of 60 to 95% and most preferably in the range of 70% to 95%, by weight, calculated to the total composition.

The suitable non limiting examples are glycerin, 1,2-propylene glycol, 1,3 propane diol, 1,3-butylene glycol, 1,2-pentylene glycol, sorbitol, panthenol and mixtures thereof. Preferred are glycerin and 1,2-propylene glycol. The most preferred is glycerin.

The composition comprises one or more amine and/or quaternary ammonium surfactant. The suitable amine or quaternary ammonium surfactants are selected from the compounds according to the general structures A, B and C 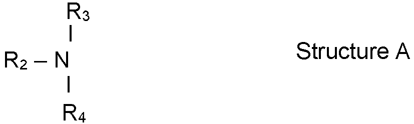 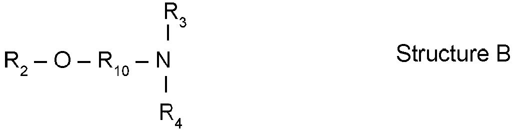 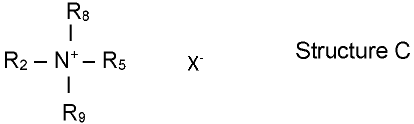
wherein R₂ is a saturated or unsaturated, branched, or straight alkyl chain with 8-24 C atoms or
R₆ CO NH (CH₂)ₙ
wherein R₆ is saturated or unsaturated, branched, or straight alkyl chain with 7-21 C atoms and n has value of 1 - 4,
   or

   R₇ CO O (CH₂)ₙ
wherein R₇ is saturated or unsaturated, branched, or straight alkyl chain with 7-21 C atoms and n has value of 1 - 4,
wherein R₁₀ is a straight alkyl chain with 1 - 4 C atoms,
R₃ and R₄ are hydrogen or unsaturated or saturated, and straight or branched alkyl chain with 1 - 4 C atoms, and
R₈, R₉ and R₅ are unsaturated or saturated, branched, or straight alkyl chain with 1 - 4 C atoms,
wherein the terms unsaturated or saturated refers to C2 to C4 alkyl chain and branched alkyl chain refers to C3 to C4 alkyl chains.

Non-limiting suitable amines are such as stearyl amine, stearoylethyl amine, stearamidopropyldimethylamine, behenamidropyl dimethyl amine, stearyl amine, palmamine, oleamine, palmitamine, stearoxypropyl dimethylamine and non-limiting suitable quaternary ammonium compounds are such as cetrimonium chloride, steartrimonium chloride, behentrimonium chloride. Preferred are stearoxypropyl dimethylamine and cetrimonium chloride.

The one or more amine and/or quaternary ammonium compounds may be comprised at a total concentration in the range of 0.1 to 10%, preferably 0.2 to 7.5% more preferably 0.25 to 5% by weight, calculated to the total composition.

The composition comprises one or more fatty alcohol. Preferably, one or more fatty alcohol is selected from the compounds according to the general structure

R₁₀-OH

wherein R₁₀ is a saturated or unsaturated, branched, or straight alkyl chain with 10 to 24C atoms.

Suitable non-limiting examples are cetyl alcohol, stearyl alcohol, cetearyl alcohol, behenyl alcohol, arachidyl alcohol, myristyl alcohol, isocetly alcohol and oleyl alcohol, and mixtures thereof.

The composition may comprise one or more fatty alcohol at a total concentration in the range of 0.1 to 10%, preferably 0.2 to 7,5% more preferably 0.25 to 5% by weight calculated to the total composition.

The composition comprises one or more glyceryl ether according to the general structure presented above selected from ethylhexyl glycerin, hexylglycerin, heptylglycerin, and methylheptylglycerin. The most preferred glyceryl ether is ethylhexyl glycerin.

The composition may comprise one or more glyceryl ether according to the general structure presented above at a total concentration in the range of 0.1 to 10%, preferably 0.2 to 7,5% more preferably 0.25 to 5% by weight, calculated to the total composition.

In an embodiment of the invention, the composition comprises one or more organic acids. The suitable non-limiting examples are citric acid, malic acid, lactic acid, glycolic acid, tartaric acid, formic acid, oxalic acid, malonic acid, succinic acid, glutaric acid, adipic acid, maleic acid, and fumaric acid. Preferred are citric acid, malic acid, lactic acid, succinic acid and maleic acid. The most preferred are citric acid, malic acid and lactic acid.

The one or more organic acid may be comprised in the compositions at a concentration in the range of 0.01 to 2% by weight calculated to the total composition.

In a further embodiment of the present invention the composition comprises one or more linear and saturated mono alcohol having 2 to 6 carbon atoms. Suitable non-limiting examples are ethanol, butanol, propanol, pentanol, hexanol, and the one or more mono alcohol may be comprised at a total concentration in the range of 1 to 30%, more preferably in the range of 2 to 25%, most preferably in the range of 5 to 25% by weight, calculated to the total composition.

The compositions of the present invention may be slightly viscous to pasty at room temperature. The compositions may be transparent or slightly cloudy to turbid. Suitably the compositions have a viscosity measured at 20°C with Brookfield DVIII spindle 5 / 5 rpm after 30 sec in the range of 1000 to 30000 mPa.s. The compositions may be confectioned in a suitable bottle equipped with a nozzle; they may as well be packed in a bottle equipped with a dispenser.

The compositions may have a pH in the range of 3.0 to 6.0, preferably 3.5 to 5.5 and more preferably 3.5 to 5.0 when diluted 1:3 by weight with water and at 20°C.

In principle there is no need to include preservatives in the compositions. Therefore, in an embodiment of the present invention the compositions comprises not more than 0.5%, preferably not more than 0.25% and most preferably not more than 0.1% antimicrobial preservatives.

The compositions may comprise any other compounds for other purposes such as fragrance, cationic polymers, ceramides, dyestuffs, thickening agents, oils including triglycerides, etc.

The following examples are to illustrate the invention but not to limit it.

### EXAMPLES

### Example 1

| **Ingredients** | **Inv. ex. 1** | **Comp. ex. 1** | **Comp. ex. 2** | **Comp. ex. 3** |
|---|---|---|---|---|
| | **[% by weight]** | | | |
| Ethylhexyl glycerin | 2.0 | - | - | 2.0 |
| Isostearyl glyceryl ether | - | - | 2.0 | - |
| Cetrimonium chloride | 2.5 | 2.5 | 2.5 | 2.5 |
| Cetearyl alcohol | 4.0 | 4.0 | 4.0 | 4.0 |
| Citric acid | 0.05 | 0.05 | 0.05 | 0.05 |
| Fragrance | 0.5 | 0.5 | 0.5 | 0.5 |
| Glycerol | ad 100.0 | | | - |
| C₁₃-C₁₅ alkane | - | | | ad 100.0 |

The above compositions were prepared by melting the fatty alcohol in glycerol or C₁₃-C₁₅ alkane at 75°C and after cooling down to 40 - 45°C temperature, the other compounds were added.

Comparative example 2 yielded a homogeneous composition immediately upon preparation, but during overnight storage the composition showed a phase separation, making the composition unsuitable for testing.

Comparative example 3 did not yield a homogeneous composition immediately upon preparation. Without being bound by any theory, the applicant believes that ethylhexyl glycerin and/or cetrimonium chloride were not soluble in C₁₃-C₁₅ alkane.

Inventive example 1 and comparative example 1 were tested as follows: In a half side test, the hair of 10 volunteers having approximately shoulder length hair and aging between 20 and 55 years was washed with a commercially available shampoo homogeneously and after dividing the hair on the head into two, the one side received the inventive composition, and the other side was applied the comparative composition. The amount applied was approximately 3 g composition each side. After leaving the composition on the hair approximately 3 min it was rinsed off with water and towel dried. The hair was evaluated by hairdressers using the scale below.
1 not satisfied at all
2 little satisfied
3 satisfied
4 well satisfied
5 very well satisfied

The following average scores were obtained.

| | Comp. ex. 1 | Inv. ex. 1 |
|---|---|---|
| Wet hair | | |
| Easy to comb | 4 | 5 |
| Softness | 2 | 4 |
| Preference | 3 | 5 |

| Dry Hair | | |
|---|---|---|
| Easy to comb | 3 | 5 |
| Softness | 3 | 5 |
| Preference | 3 | 5 |

From the above, it is beyond any doubt the composition according to the invention has better conditioning effect on hair than the comparative composition.

### Example 2

| **Ingredients** | **Inv. ex. 2** | **Comp. ex. 4** |
|---|---|---|
| | **[% by weight]** | |
| Ethylhexyl glycerin | 2.0 | - |
| Behenamidopropyl Dimethylamine | 2.5 | 2.5 |
| Cetearyl alcohol | 4.0 | 4.0 |
| Citric acid | 1.5 | 1.5 |
| Fragrance | 0.5 | 0.5 |
| Glycerol | ad 100.0 | |

The above compositions were prepared by melting the fatty alcohol in glycerol at 75°C and after cooling down to room temperature, the other compounds were added.

The half side test and the evaluation were done in the same way as in Example 1.

The following scores were obtained.

| | Comp. ex. 4 | Inv. ex. 2 |
|---|---|---|
| Wet hair | | |
| Easy to comb | 3 | 5 |
| Softness | 3 | 4 |
| Preference | 2 | 5 |

| Dry Hair | | |
|---|---|---|
| Easy to comb | 2 | 4 |
| Softness | 2 | 4 |
| Preference | 3 | 5 |

From the above, it is beyond any doubt the composition according to the invention has better conditioning effect on hair than the comparative composition.

### Example 3

| **Ingredients** | **Inv. ex. 3** | **Comp. ex. 4** | **Comp. ex. 5** |
|---|---|---|---|
| | **[% by weight]** | | |
| Ethylhexyl glycerin | 2.0 | - | 2.0 |
| Cetrimonium chloride | 2.5 | 2.5 | 2.5 |
| Cetearyl alcohol | 4.0 | 4.0 | 4.0 |
| Citric acid | 0.05 | 0.05 | 0.05 |
| Fragrance | 0.5 | 0.5 | 0.5 |
| Glycerol | 70.0 | 72.0 | - |
| Ethanol | Ad 100.0 | Ad 100.0 | - |
| Water | - | - | Ad 100.0 |

Inventive example 3 was prepared in the same way as Example 1. The composition had a good conditioning effect on human hair which was confirmed in a comparative test against a comparative example 4.

Comparative example 5 was prepared as an aqueous composition by melting the cetearyl alcohol in the presence of cetrimonium chloride in water and adding other substances afterwards. A comparative test was carried out against inventive example 1 as described under Example 1. Evaluation was also done in the same way as described under Example 1. The following results were obtained.

| | Comparative | Inventive |
|---|---|---|
| Wet hair | | |
| Easy to comb | 2 | 5 |
| Softness | 2 | 4 |
| Preference | 2 | 5 |

| Dry Hair | | |
|---|---|---|
| Easy to comb | 3 | 5 |
| Softness | 2 | 4 |
| Preference | 2 | 5 |

From the above results, it is beyond any doubt the anhydrous inventive example 3 conditioned hair much better than the above comparative example 5. It was especially pointed out by the testing of the hairdressers that the side with inventive example 3 was felt far softer than the side treated with the comparative example 5. The side treated with the inventive example was the preferred side and received the highest score.

## Claims

1. A substantially anhydrous composition for keratin fibers especially for human hair **characterized in that** it comprises
- one or more polyol other than the glyceryl ether,
- one or more amine and/or quaternary ammonium surfactant,
- one or more fatty alcohol,
- one or more glyceryl ether according to the general structure 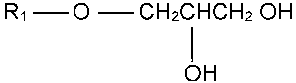
wherein R₁ is straight or branched, saturated or unsaturated alkyl chain with 4 to 18 C atoms,
wherein the composition comprises water not more than 10%, preferably 7.5%, more preferably 5% and most preferably 2.5% by weight, calculated to the total weight of the composition,
wherein the composition comprises the one or more polyol at a concentration
of at least 40% by weight, calculated to the total weight of the composition, and
wherein the one or more glyceryl ether according to the above general structure is selected from ethylhexyl glycerin, hexylglycerin, heptylglycerin, methylheptylglycerin.

2. The composition according to claim 1 **characterized in that** the one or more amine and/or quaternary ammonium surfactant is selected from the compounds according to the general structures A, B, or C, 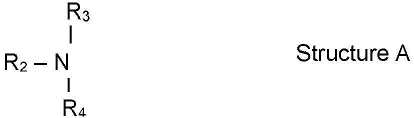 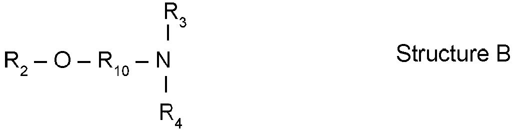 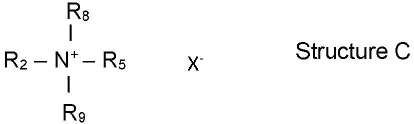
wherein R₂ is a saturated or unsaturated, branched, or straight alkyl chain with 8-24 C atoms or
R₆ CO NH (CH₂)ₙ
wherein R₆ is saturated or unsaturated, branched, or straight alkyl chain with 7-21 C atoms and n has value of 1 - 4,
or
R₇ CO O (CH₂)ₙ
wherein R₇ is saturated or unsaturated, branched, or straight alkyl chain with 7-21 C atoms and n has value of 1 - 4,
wherein R₁₀ is a straight alkyl chain with 1 - 4 C atoms,
R₃ and R₄ are hydrogen or unsaturated or saturated, and straight or branched alkyl chain with 1 - 4 C atoms, and
R₈, R₉ and R₅ are unsaturated or saturated, branched, or straight alkyl chain with 1 - 4 C atoms,
wherein the terms unsaturated or saturated refers to C2 to C4 alkyl chain and branched alkyl chain refers to C3 to C4 alkyl chains.

3. The composition according to claims 1 and/or 2 **characterized in that** one or more fatty alcohol is selected from the compounds according to the general structure
R₁₀-OH
wherein R₁₀ is a saturated or unsaturated, branched, or straight alkyl chain with 10 to 24C atoms and preferably selected from cetyl alcohol, stearyl alcohol, cetearyl alcohol, behenyl alcohol, arachidyl alcohol, myristyl alcohol, isocetly alcohol and oleyl alcohol, and mixures thereof.

4. The composition according to any of the preceding claims **characterized in that** the composition comprises one or more polyol at a concentration of at least 50%, more preferably 60 to 90% and most preferably 70% to 80%, by weight, calculated to the total weight of the composition.

5. The composition according to any of the preceding claims **characterized in that** one or more polyol is selected form glycerol, 1,2-propylene glycol, 1,3 propane diol, 1,3-butylene glycol, 1,2-pentylene glycol, sorbitol, panthenol, and mixtures thereof.

6. The composition according to any of the preceding claims **characterized in that** the composition comprises one or more amine or quaternary ammonium surfactant at a total concentration in the range of 0.1% to 10% by weight, preferably 0.2% to 7.5% by weight, more preferably 0.25% to 5% by weight, calculated to the total weight of the composition.

7. The composition according to any of the preceding claims **characterized in that** the composition comprises one or more fatty alcohols at a concentration in the range of 0.1% to 10% by weight, preferably 0.2% to 7.5% by weight, more preferably 0.25% to 5%, by weight calculated to the total weight of the composition.

8. The composition according to any of the preceding claims **characterized in that** the composition comprises one or more glycerin ether according to the above general structure at a total concentration in the range of 0.1% to 10% by weight, preferably 0.2% to 7.5% by weight, more preferably 0.25% to 5% by weight, calculated to the total weight of the composition.

9. The composition according to any of the preceding claims **characterized in that** the one or more glyceryl ether according to the general structure above is ethylhexyl glycerin.

10. The composition according to any of the preceding claims **characterized in that** the one or more amine or quaternary ammonium surfactant is selected from stearyl amine, stearoylethyl amine, stearamidopropyldimethylamine, behenamidropyl dimethyl amine, stearyl amine, palmamine, oleamine, palmitamine, stearoxypropyl dimethylamine, cetrimonium chloride, steartrimonium chloride, behentrimonium chloride, preferably said surfactant is selected from stearoxypropyl dimethylamine and cetrimonium chloride.

11. The composition according to any of the preceding claims **characterized in that** it comprises one or more organic acids, preferably said organic acids are selected from citric acid, malic acid, lactic acid, glycolic acid, tartaric acid, formic acid, oxalic acid, malonic acid, succinic acid, glutaric acid, adipic acid, maleic acid, and fumaric acid, further preferably the total concentration of the one or more organic acids is in the range of 0.01% to 2% by weight, calculated to the total weight of the composition.

12. The composition according to any of the preceding claims **characterized in that** the composition has a pH in the range of 3.0 to 6.0, preferably 3.5 to 5.5 and more preferably 3.5 to 5.0, when diluted 1:3 by weight with water and at 20°C.

13. The composition according to any of the preceding claims **characterized in that** it comprises one or more linear and saturated mono alcohol having 2 to 6 carbon atoms, preferably said alcohol is selected from ethanol, butanol, propanol, pentanol, hexanol, and more preferably the total concentration of said alcohols is in the range of 1% to 30% by weight, more preferably in the range of 2% to 25% by weight, still more preferably in the range of 5% to 25% by weight, calculated to the total weight of the composition.

14. A method of conditioning keratin fibers especially human hair, comprising the following steps:
(i) Treating the keratin fibers with an aqueous composition, preferably a cleansing composition, and then, when the keratin fibers are wet,
(ii) Applying the composition according to any of the claims 1 to 13 onto the keratin fibers and leaving it for a time period in the range of 30 s to 10 min, then
(iii) Rinsing off the composition from the keratin fibers, and then
(iv) Towel drying the keratin fibers.

15. Kit for hair comprising two or more components wherein one of the components is a composition according to claims 1 to 13.

## Patentansprüche

1. Im Wesentlichen wasserfreie Zusammensetzung für keratinische Fasern, insbesondere für menschliches Haar, **dadurch gekennzeichnet, dass** sie Folgendes umfasst
- ein oder mehrere andere Polyole als Glycerylether,
- ein oder mehrere Amin- und/oder quartäre Ammonium-Tenside,
- ein oder mehrere Fettalkohole,
- ein oder mehrere Glycerylether gemäß der allgemeinen Struktur 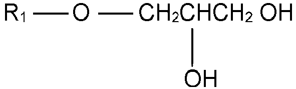
wobei R₁ eine geradkettige oder verzweigte, gesättigte oder ungesättigte Alkylkette mit 4 bis 18 C-Atomen ist,
wobei die Zusammensetzung nicht mehr als 10 Gew.-%, vorzugsweise 7,5 Gew.-%, noch bevorzugter 5 Gew.-% und am meisten bevorzugt 2,5 Gew.-%, berechnet auf das Gesamtgewicht der Zusammensetzung, Wasser umfasst,
wobei die Zusammensetzung das eine oder die mehreren Polyol(e) in einer Konzentration von mindestens 40 Gew.-%, berechnet auf das Gesamtgewicht der Zusammensetzung, umfasst, und
wobei der eine oder die mehreren Glycerylether gemäß der obigen allgemeinen Struktur aus Ethylhexylglycerin, Hexylglycerin, Heptylglycerin, Methylheptylglycerin ausgewählt sind.

2. Zusammensetzung nach Anspruch 1, **dadurch gekennzeichnet, dass** das eine oder die mehreren Amin- und/oder quaternären Ammoniumtenside ausgewählt sind aus den Verbindungen gemäß den allgemeinen Strukturen A, B oder C, 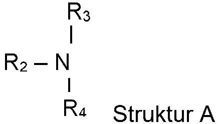 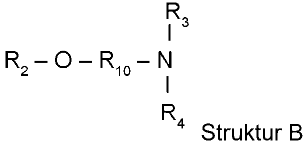 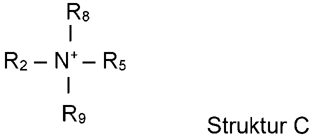
wobei R₂ eine gesättigte oder ungesättigte, verzweigte oder geradkettige Alkylkette mit 8-24 C-Atomen ist oder
R₆ CO NH (CH₂)ₙ
wobei R₆ eine gesättigte oder ungesättigte, verzweigte oder geradkettige Alkylkette mit 7-21 Kohlenstoffatomen ist und n einen Wert von 1 - 4 aufweist,
oder
R₇ CO O (CH₂)ₙ
wobei R₇ eine gesättigte oder ungesättigte, verzweigte oder geradkettige Alkylkette mit 7-21 Kohlenstoffatomen ist und n einen Wert von 1 - 4 aufweist,
wobei R₁₀ eine geradkettige Alkylkette mit 1 - 4 C-Atomen ist,
R₃ und R₄ Wasserstoff oder eine ungesättigte oder gesättigte, und geradkettige oder verzweigte Alkylkette mit 1 - 4 C-Atomen sind, und
R₈, R₉ und R₅ ungesättigte oder gesättigte, verzweigte oder geradkettige Alkylketten mit 1 - 4 C-Atomen sind,
wobei sich die Begriffe "ungesättigt" oder "gesättigt" auf C2- bis C4-Alkylketten beziehen und "verzweigte Alkylketten" sich auf C3- bis C4-Alkylketten beziehen.

3. Zusammensetzung nach Anspruch 1 und/oder 2, **dadurch gekennzeichnet, dass** ein oder mehrere Fettalkohole aus den Verbindungen gemäß der allgemeinen Struktur
R₁₀-OH
ausgewählt sind
wobei R₁₀ eine gesättigte oder ungesättigte, verzweigte oder geradkettige Alkylkette mit 10 bis 24 C-Atomen ist und vorzugsweise aus Cetylalkohol, Stearylalkohol, Cetearylalkohol, Behenylalkohol, Arachidylalkohol, Myristylalkohol, Isocetylalkohol und Oleylalkohol sowie Mischungen dieser ausgewählt ist.

4. Zusammensetzung nach einem der vorstehenden Ansprüche, **dadurch gekennzeichnet, dass** die Zusammensetzung ein oder mehrere Polyole in einer Konzentration von mindestens 50 %, vorzugsweise 60 bis 90 % und am meisten bevorzugt 70 % bis 80 Gew.-%, berechnet auf das Gesamtgewicht der Zusammensetzung, umfasst.

5. Zusammensetzung nach einem der vorstehenden Ansprüche, **dadurch gekennzeichnet, dass** ein oder mehrere Polyole aus Glycerin, 1,2-Propylenglykol, 1,3-Propandiol, 1,3-Butylenglykol, 1,2-Pentylenglykol, Sorbit, Panthenol und Mischungen dieser ausgewählt sind.

6. Zusammensetzung nach einem der vorstehenden Ansprüche, **dadurch gekennzeichnet, dass** die Zusammensetzung ein oder mehrere Amin- oder quaternäre Ammoniumtenside in einer Gesamtkonzentration im Bereich von 0,1 % bis 10 Gew.-%, vorzugsweise 0,2 % bis 7,5 Gew.-%, noch bevorzugter 0,25 % bis 5 Gew.-%, berechnet auf das Gesamtgewicht der Zusammensetzung, umfasst.

7. Zusammensetzung nach einem der vorstehenden Ansprüche, **dadurch gekennzeichnet, dass** die Zusammensetzung einen oder mehrere Fettalkohole in einer Konzentration im Bereich von 0,1 % bis 10 Gew.-%, vorzugsweise 0,2 % bis 7,5 Gew.-%, noch bevorzugter 0,25 % bis 5 Gew.-%, berechnet auf das Gesamtgewicht der Zusammensetzung, umfasst.

8. Zusammensetzung nach einem der vorstehenden Ansprüche, **dadurch gekennzeichnet, dass** die Zusammensetzung einen oder mehrere Glycerinether gemäß der obigen allgemeinen Struktur in einer Gesamtkonzentration im Bereich von 0,1 % bis 10 Gew.-%, vorzugsweise 0,2 % bis 7,5 Gew.-%, noch bevorzugter 0,25 % bis 5 Gew.-%, berechnet auf das Gesamtgewicht der Zusammensetzung, umfasst.

9. Zusammensetzung nach einem der vorstehenden Ansprüche, **dadurch gekennzeichnet, dass** der eine oder die mehreren Glycerylether gemäß der obigen allgemeinen Struktur Ethylhexylglycerin sind.

10. Zusammensetzung nach einem der vorstehenden Ansprüche, **dadurch gekennzeichnet, dass** das eine oder die mehreren Amin- oder quaternären Ammoniumtenside aus Stearylamin, Stearoylethylamin, Stearamidopropyldimethylamin, Behenamidropyldimethylamin, Stearylamin, Palmamin, Oleamin, Palmitamin, Stearoxypropyldimethylamin, Cetrimoniumchlorid, Steartrimoniumchlorid, Behentrimoniumchlorid ausgewählt ist/sind, wobei das Tensid vorzugsweise aus Stearoxypropyldimethylamin und Cetrimoniumchlorid ausgewählt ist.

11. Zusammensetzung nach einem der vorstehenden Ansprüche, **dadurch gekennzeichnet, dass** diese eine oder mehrere organische Säuren umfasst, wobei die organischen Säuren vorzugsweise aus Zitronensäure, Apfelsäure, Milchsäure, Glykolsäure, Weinsäure, Ameisensäure, Oxalsäure, Malonsäure, Bernsteinsäure, Glutarsäure, Adipinsäure, Maleinsäure und Fumarsäure ausgewählt sind, wobei die Gesamtkonzentration der einen oder mehreren organischen Säuren vorzugsweise im Bereich von 0,01 Gew.-% bis 2 Gew.-%, berechnet auf das Gesamtgewicht der Zusammensetzung, liegt.

12. Zusammensetzung nach einem der vorstehenden Ansprüche, **dadurch gekennzeichnet, dass** die Zusammensetzung einen pH-Wert im Bereich von 3,0 bis 6,0, vorzugsweise 3,5 bis 5,5 und noch bevorzugter 3,5 bis 5,0 aufweist, wenn sie im Gewichtsverhältnis 1:3 mit Wasser verdünnt wird und eine Temperatur von 20 °C aufweist.

13. Zusammensetzung nach einem der vorstehenden Ansprüche, **dadurch gekennzeichnet, dass** diese einen oder mehrere lineare und gesättigte Monoalkohole mit 2 bis 6 Kohlenstoffatomen umfasst, wobei der Alkohol vorzugsweise aus Ethanol, Butanol, Propanol, Pentanol, Hexanol ausgewählt ist, und wobei die Gesamtkonzentration dieser Alkohole vorzugsweise im Bereich von 1 % bis 30 Gew.-%, bevorzugter im Bereich von 2 % bis 25 Gew.-%, noch bevorzugter im Bereich von 5 % bis 25 Gew.-%, berechnet auf das Gesamtgewicht der Zusammensetzung, liegt.

14. Verfahren zur Konditionierung von keratinische Fasern, insbesondere von menschlichem Haar, umfassend die folgenden Schritte:
(i) Behandeln der keratinische Fasern mit einer wässrigen Zusammensetzung, vorzugsweise einer Reinigungszusammensetzung, und dann, wenn die keratinische Fasern nass sind,
(ii) Auftragen der Zusammensetzung nach einem der Ansprüche 1 bis 13 auf die keratinische Fasern und Einwirkenlassen für einen Zeitraum im Bereich von 30 s bis 10 min, dann
(iii) Abspülen der Zusammensetzung von den keratinische Fasern, und dann
(iv) Trocknen der keratinische Fasern mit einem Handtuch.

15. Set für Haare, umfassend zwei oder mehr Komponenten, wobei eine der Komponenten eine Zusammensetzung gemäß den Ansprüchen 1 bis 13 ist.

## Revendications

1. Composition sensiblement anhydre pour fibres kératiniques, en particulier pour cheveux humains, **caractérisée en ce qu'**elle comprend
- un ou plusieurs polyols autres que l'éther de glycéryle,
- un ou plusieurs tensioactifs d'amine et/ou d'ammonium quaternaire,
- un ou plusieurs alcools gras,
- un ou plusieurs éthers de glycéryle selon la structure générale 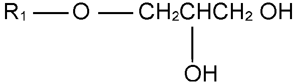
dans laquelle R₁ est une chaîne d'alkyle droite ou ramifiée, saturée ou insaturée avec 4 à 18 atomes de C,
dans laquelle la composition comprend de l'eau à hauteur de pas plus de 10 %, préférablement à 7,5 %, plus préférablement à 5 % et le plus préférentiellement à 2,5 % en poids, calculée par rapport au poids total de la composition,
dans laquelle la composition comprend les
un ou plusieurs polyols à une concentration
d'au moins 40 % en poids, calculée par rapport au poids total de la composition, et dans laquelle les un ou plusieurs éthers de glycéryle selon la structure générale ci-dessus sont sélectionnés parmi la glycérine d'éthylhexyle, l'hexylglycérine, l'heptylglycérine, la méthylheptylglycérine.

2. Composition selon la revendication 1 **caractérisée en ce que** les un ou plusieurs tensioactifs d'amine et/ou d'ammonium quaternaire sont sélectionnés parmi les composés selon les structures générales A, B ou C, 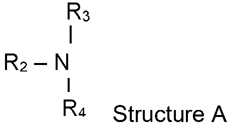 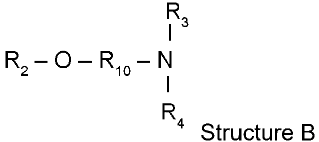 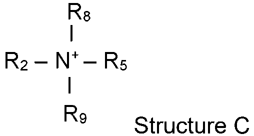
dans laquelle R₂ est une chaîne d'alkyle droite ou ramifiée, saturée ou insaturée avec 8 à 24 atomes de C ou
R₆ CO NH (CH₂)ₙ
dans laquelle R₆ est une chaîne d'alkyle droite ou ramifiée, saturée ou insaturée avec 7 à 21 atomes de C et n présente une valeur de 1 à 4,
ou
R₇ CO O (CH₂)ₙ
dans laquelle R₇ est une chaîne d'alkyle droite ou ramifiée, saturée ou insaturée avec 7 à 21 atomes de C et n présente une valeur de 1 à 4,
dans laquelle R₁₀ est une chaîne d'alkyle droite avec 1 à 4 atomes de C,
R₃ et R₄ sont de l'hydrogène ou une chaîne d'alkyle droite ou ramifiée, saturée ou insaturée avec 1 à 4 atomes de C, et
R₈, R₉ et R₅ sont une chaîne d'alkyle droite ou ramifiée, saturée ou insaturée avec 1 à 4 atomes de C,
dans laquelle les termes insaturé ou saturé se réfèrent à une chaîne d'alkyle en C2 à C4 et le terme chaîne d'alkyle ramifiée se réfère à des chaînes d'alkyle en C3 à C4.

3. Composition selon les revendications 1 et/ou 2 **caractérisée en ce qu'**un ou plusieurs alcools gras sont sélectionnés parmi les composés selon la structure générale
R₁₀-OH
dans laquelle R₁₀ est une chaîne d'alkyle droite ou ramifiée, saturée ou insaturée avec 10 à 24 atomes de C et préférablement sélectionné parmi alcool cétylique, alcool stéarylique, alcool cétéarylique, alcool béhénylique, alcool arachidylique, alcool myristylique, alcool isocétylique et alcool oléylique, et des mélanges de ceux-ci.

4. Composition selon l'une quelconque des revendications précédentes **caractérisée en ce que** la composition comprend un ou plusieurs polyols à une concentration d'au moins 50 %, plus préférablement de 60 à 90 % et le plus préférentiellement de 70 % à 80 % en poids, calculée par rapport au poids total de la composition.

5. Composition selon l'une quelconque des revendications précédentes **caractérisée en ce qu'**un ou plusieurs polyols sont sélectionnés parmi glycérol, 1,2-propylène glycol, 1,3 propane diol, 1,3-butylène glycol, 1,2-pentylène glycol, sorbitol, panthénol, et des mélanges de ceux-ci.

6. Composition selon l'une quelconque des revendications précédentes **caractérisée en ce que** la composition comprend un ou plusieurs tensioactifs d'amine ou d'ammonium quaternaire à une concentration totale comprise dans la plage de 0,1 % à 10 % en poids, préférablement de 0,2 % à 7,5 % en poids, plus préférablement de 0,25 % à 5 % en poids, calculée par rapport au poids total de la composition.

7. Composition selon l'une quelconque des revendications précédentes **caractérisée en ce que** la composition comprend un ou plusieurs alcools gras à une concentration comprise dans la plage de 0,1 % à 10 % en poids, préférablement de 0,2 % à 7,5 % en poids, plus préférablement de 0,25 % à 5 %, en poids, calculée par rapport au poids total de la composition.

8. Composition selon l'une quelconque des revendications précédentes **caractérisée en ce que** la composition comprend un ou plusieurs éthers de glycérine selon la structure générale ci-dessus à une concentration totale comprise dans la plage de 0,1 % à 10 % en poids, préférablement de 0,2 % à 7,5 % en poids, plus préférablement de 0,25 % à 5 %, en poids, calculée par rapport au poids total de la composition.

9. Composition selon l'une quelconque des revendications précédentes **caractérisée en ce que** les un ou plusieurs éthers de glycéryle selon la structure générale ci-dessus est la glycérine d'éthylhexyle.

10. Composition selon l'une quelconque des revendications précédentes **caractérisée en ce que** les un ou plusieurs tensioactifs d'amine ou d'ammonium quaternaire sont sélectionnés parmi amine de stéaryle, amine de stéaroyléthyle, stéaramidopropyldiméthylamine, amine diméthylique de béhénamidropyle, amine de stéaryle, palmamine, oléamine, palmitamine, diméthylamine de stéaroxypropyle, chlorure de cétrimonium, chlorure de stéartrimonium, chlorure de béhentrimonium, préférablement ledit tensioactif est sélectionné parmi diméthylamine de stéaroxypropyle et chlorure de cétrimonium.

11. Composition selon l'une quelconque des revendications précédentes **caractérisée en ce qu'**elle comprend un ou plusieurs actives organiques, préférablement lesdits acides organiques sont sélectionnés parmi acide citrique, acide malique, acide lactique, acide glycolique, acide tartrique, acide formique, acide oxalique, acide malonique, acide succinique, acide glutarique, acide adipique, acide maléique et acide fumarique, en outre préférablement la concentration totale des un ou plusieurs acides organiques est comprise dans la plage de 0,01 % à 2 % en poids, calculée par rapport au poids total de la composition.

12. Composition selon l'une quelconque des revendications précédentes **caractérisée en ce que** la composition présente un pH dans la plage de 3,0 à 6,0, préférablement de 3,5 à 5,5 et plus préférablement de 3,5 à 5,0, lorsqu'elle est diluée à un rapport de 1:3 en poids avec de l'eau et à 20 °C.

13. Composition selon l'une quelconque des revendications précédentes **caractérisée en ce qu'**elle comprend un ou plusieurs monoalcools linéaires et saturés présentant 2 à 6 atomes de carbone, préférablement ledit alcool est sélectionné parmi éthanol, butanol, propanol, pentanol, hexanol, et plus préférablement la concentration totale desdits alcools est comprise dans la plage de 1 % à 30 % en poids, plus préférablement dans la plage de 2 % à 25 % en poids, de façon encore plus préférée dans la plage de 5 % à 25 % en poids, calculée par rapport au poids total de la composition.

14. Procédé de revitalisation de fibres kératiniques, en particulier de cheveux humains, comprenant les étapes suivantes consistant à :
(i) traiter les fibres kératiniques avec une composition aqueuse, préférablement une composition de nettoyage, puis, lorsque les fibres kératiniques sont humides,
(ii) appliquer la composition selon l'une quelconque des revendications 1 à 13 sur les fibres kératiniques et la laisser reposer pendant une durée allant de 30 s à 10 min, puis
(iii) rincer la composition des fibres kératiniques, puis
(iv) sécher les fibres kératiniques à l'aide d'une serviette.

15. Kit pour cheveux comprenant deux composants ou plus, dans lequel l'un des composants est une composition selon les revendications 1 à 13.
